# EUROPÄISCHE PATENTSCHRIFT

(11) **EP 0 393 354 B1**
(45) Veröffentlichungstag und Bekanntmachung des Hinweises auf die Patenterteilung: **13.01.1993**
(21) Anmeldenummer: 90104957.7
(22) Anmeldetag: 16.03.1990
(51) Int. Cl.: A61M 5/142, A61M 5/168, F04B 43/12

(54) **Medizinisches Pumpgerät**
Medical pump appliance
Outillage de pompe médical

(30) Priorität: 15.04.1989 DE 3912405
(43) Veröffentlichungstag der Anmeldung: 24.10.1990
(73) Patentinhaber: B. Braun Melsungen AG, 34209 Melsungen (DE)
(72) Erfinder: Mosebach, Wolfgang, D-3509 Dagobertshausen (DE); Heitmeier, Rolf, D-3507 Baunatal (DE); Knuth, Reinhard, D-3508 Melsungen (DE)
(74) Vertreter: Selting, Günther, Dipl.-Ing.

(56) Entgegenhaltungen:
- EP-A- 0 069 350
- DE-A- 3 018 641
- DE-A- 3 202 251

## Beschreibung

Die Erfindung betrifft ein medizinisches Pumpgerät der im Oberbegriff des Patentanspruchs 1 angegebenen Art. Solche eine Pumpgerät zeigen beispielsweise die DE-A-3 018 641 oder die DE-A-3 202 251.

Im medizinischen Bereich werden medizinische Pumpgeräte dazu benutzt, dem Körper eines Patienten eine Infusionsflüssigkeit in exakt dosierter Rate zuzuführen. Wegen der hohen Sterilitätsanforderungen werden in medizinischen Pumpgeräten Schlauchpumpen eingesetzt, bei denen zum Vortrieb der Flüssigkeit ein Pumpenschlauch fortlaufend abgequetscht wird. Der Pumpenschlauch ist ein Einmalschlauch, der nach seiner Benutzung fortgeworfen wird. Er sollte daher möglichst kostengünstig herstellbar sein. Entsprechendes gilt auch für andere Pumpgeräte, z.B. Blutpumpen, die dem Körper des Patienten Blut entziehen oder dem Körper Blut zuführen. Auch hier ist die Erzielung und Einhaltung einer gewünschten Förderrate von großer Wichtigkeit.

Die Förderrate einer Schlauchpumpe hängt wesentlich von der Maßgenauigkeit des Pumpenschlauchs ab. Bisher wurde zur genauen Erzielung gewünschter Förderraten besonderer Wert auf eine maßgenaue Herstellung des Pumpenschlauches gelegt, wobei solche Pumpenschläuche, deren Fördervolumen außerhalb gewisser Toleranzgrenzen lag, als unbrauchbar aussortiert wurden. Innerhalb der Toleranzgrenzen haben die verwendeten Pumpenschläuche hierbei aber immer noch erhebliche Abweichungen des Fördervolumens. Wenn man die Toleranzgrenzen eng wählt, entsteht viel Ausschuß, und wenn man sie weit wählt, entstehen große Abweichungen der Förderrate von der Soll-Förderrate.

Der Erfindung liegt die Aufgabe zugrunde, ein medizinisches Pumpgerät der im Oberbegriff des Patentanspruchs 1 angegebenen Art zu schaffen, bei dem ohne besondere Maßhaltigkeitsanforderungen an den Pumpenschlauch eine genaue Einhaltung der gewünschten Förderrate erfolgt.

Die Lösung dieser Aufgabe erfolgt erfindungsgemäß mit den im kennzeichnenden Teil des Patentanspruchs 1 angegebenen Merkmalen.

Bei dem erfindungsgemäßen Pumpgerät sind die einzelnen Pumpenschläuche jeweils mit einer Kodierung versehen, die ein Maß über das individuelle Fördervolumen dieses Schlauchs abgibt bzw. die Abweichungen von dem Soll-Fördervolumen darstellt. Auf diese Weise erhält jeder Schlauch Angaben über sein individuelles Fördervolumen. Diese Angaben werden von einer Lesevorrichtung des Pumpgerätes gelesen. Die Steuersignale, die die Antriebsgeschwindigkeit der Schlauchpumpe bestimmen, werden durch die gelesenen Kodiersignale korrigiert, so daß die tatsächliche Antriebsgeschwindigkeit der Pumpe auf den verwendeten Schlauch derart abgestimmt wird, daß die Ist-Förderrate der eingestellten Soll-Förderrate mit hoher Genauigkeit entspricht, unabhängig von der Abweichung des Fördervolumens vom Soll-Fördervolumen bei dem betreffenden Pumpenschlauch.

Die Kodierung kann auf einem am Schlauch angebrachten Kodierungsträger angeordnet sein oder auch am Schlauch selbst, beispielsweise in Form von maschinenlesbaren Ringen oder anderen Zeichen.

Gemäß einer vorteilhaften Weiterbildung der Erfindung weist der Schlauch eine Fixiervorrichtung auf, die seine Anbringung ausschließlich in einer definierten Position am Gehäuse des Pumpgerätes zuläßt. In dieser definierten Position wird die Kodierung von der Lesevorrichtung erfaßt. Das Lesen der Kodierung erfolgt selbsttätig nach dem Einsetzen des Schlauchs in das Gehäuse und vor der Inbetriebnahme der Pumpe. Die Bedienungsperson braucht keine besonderen Maßnahmen durchzuführen und das Einlesen des Korrekturcodes in die Lesevorrichtung kann nicht vergessen werden.

Die Erfindung ist mit Vorteil bei einem mikroprozessorgesteuerten Infusionsgerät anwendbar, wo lediglich eine entsprechende Programmierung des Mikroprozessors erfolgen muß, um die Korrektur der Antriebsgeschwindigkeit der Pumpe zur Erzielung einer bestimmten Förderrate durchzuführen. Die Erfindung kann aber auch für andere medizinische Pumpgeräte benutzt werden, beispielsweise für mikroprozessorgesteuerte Blutpumpen, Dialysepumpen u.dgl.

Im folgenden wird unter Bezugnahme auf die Zeichnungen ein Ausführungsbeispiel der Erfindung näher erläutert.

Es zeigen:

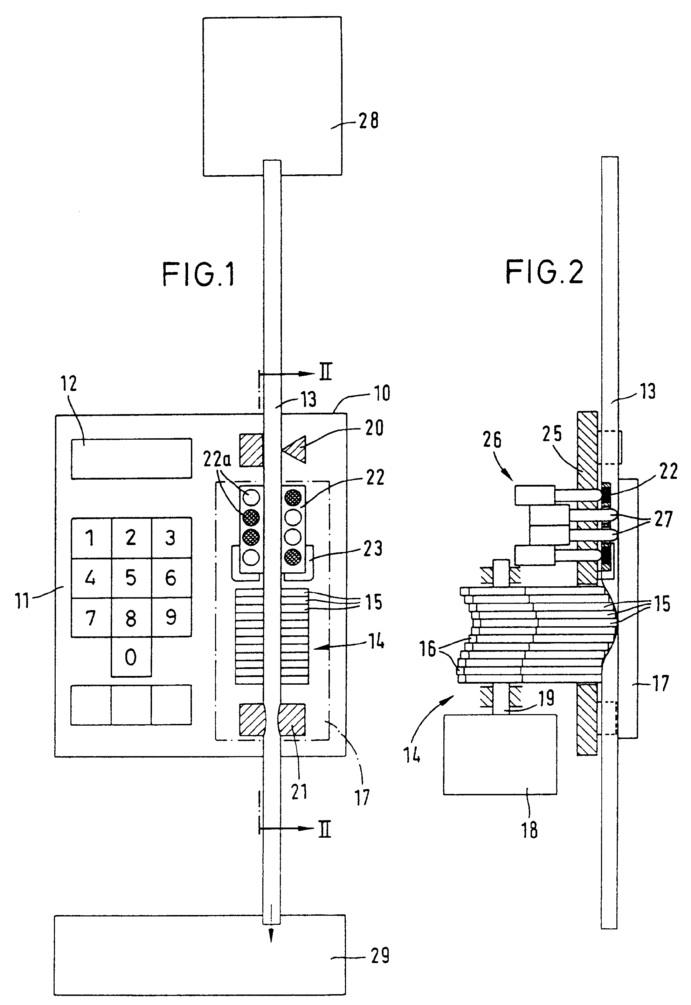

Fig. 1 eine Frontansicht eines Infusionsgerätes,
Fig. 2 eine schematische Darstellung eines Vertikalschnitts, im wesentlichen entlang der Linie II-II von Fig. 1 und
Fig. 3 ein Blockschaltbild der Steuerung des Infusionsgerätes.

Das dargestellte Infusionsgerät weist ein Gehäuse 10 auf, an dem eine Eingabetastatur 11 für die Eingabe der Förderrate und anderer Daten sowie eine Anzeigevorrichtung 12 vorgesehen sind. An der Frontseite des Gehäuses 10 befindet sich ein vertikaler Kanal, in den der Pumpenschlauch 13 der Schlauchpumpe 14 eingesetzt werden kann. Die Schlauchpumpe ist bei dem vorliegenden Ausführungsbeispiel eine Fingerpumpe, die zahlreiche Pumpenfinger 15 aufweist, welche von Nocken 16 angetrieben sind, die auf einer gemeinsamen Antriebswelle 19 sitzen. Die Antriebswelle 19 wird von einem Motor angetrieben und die Nocken 16 sind derart ausgebildet, daß sie die Pumpenfinger 15 periodisch quer zum Pumpenschlauch 13 bewegen. Alle Pumpenfinger 15 befinden sich in unterschiedlichen Phasen ihrer Linearbewegung, so daß die von den Pumpenfingern bewirkte Abquetschung des Schlauchs 13 sich linear in Schlauchrichtung bewegt. Dabei wird der Schlauch von einem plattenförmigen Gegenlager 17 abgestützt, das nach Art einer Tür vor dem Gehäuse 10 angebracht ist.

Der Schlauch 13 erstreckt sich in dem vertikalen Kanal, der in der Frontwand des Gehäuses vorgesehen ist und der auf einem Teil seiner Länge durch das Gegenlager 17 bedeckt ist. Oberhalb der Pumpe 14 findet sich eine Sicherheitsklemme 20, die zum Absperren des Schlauchs betätigt werden kann, und unterhalb der Schlauchpumpe 14 ist ein Drucksensor 21 zur Messung des Innendrucks des Schlauchs angeordnet.

An dem Schlauch 13 ist eine Kodierplatte 22 angebracht, die die Kodierung 22a trägt. Die Kodierung besteht bei dem vorliegenden Ausführungsbeispiel aus gelochten und ungelochten Bereichen der Kodierplatte 22. Die Kodierplatte weist zwei Flügel auf, die sich nach entgegengesetzten Seiten des Schlauchs erstrecken und dort neben dem Schlauch 13 die Kodierungen 22a tragen. Im vorliegenden Fall sind zwei vertikale Spalten mit jeweils vier Kodierpositionen vorhanden, so daß insgesamt 2⁸ = 256 unterschiedliche Kodeworte realisiert werden können.

Am Gehäuse 10 ist ferner eine Fixiervorrichtung 23 vorgesehen, die sicherstellt, daß der Schlauch nur in definierter Position eingesetzt werden kann und daß sich dabei die Kodierplatte 22 ebenfalls in einer definierten Position befindet. Die Fixiervorrichtung 23 besteht bei dem vorliegenden Ausführungsbeispiel aus zwei L-förmigen Bügeln, zwischen denen der Schlauch hindurchgeht und die Auflager sowie Seitenlager für die Kodierplatte 22 bilden. Die Kodierplatte 22 bildet gleichzeitig einen Aufhänger, um den Schlauch 13 an dem Gehäuse aufzuhängen. Das plattenförmige Gegenlager 17, das den Schlauch 13 gegen die Kraft der Pumpenfinger 15 abstützt, erstreckt sich nach oben hin bis über den Bereich der Kodierplatte 22 hinaus, so daß die Kodierplatte von dem Gegenlager 17 überdeckt wird. Auf diese Weise ist sichergestellt, daß die Kodierplatte 22 bei geschlossenem Gegenlager 17 gegen die den Boden des Schlauchkanals bildende Gehäusewand 25 anliegt.

An der Gehäusewand 25 ist die Lesevorrichtung 26 angebracht. Diese weist für jede Kodierstelle einen Fühler 27 auf, der bei dem vorliegenden Ausführungsbeispiel ein mechanischer Taststift ist. Sämtliche Fühler 27 sind in Richtung auf die Kodierplatte 22 vorgespannt und wenn eine Kodierposition ein Loch ist, so durchdringt der betreffende Fühler 27 dieses Loch. Ist die Kodierposition dagegen geschlossen, so stößt der Fühler gegen diese Kodierposition. Aufgrund der Fühlerstellungen werden Kodiersignale erzeugt, wobei jedes Kodiersignal aus einer der Anzahl der Kodierstellen entsprechenden Bit-Zahl besteht.

Nach der Herstellung des Pumpenschlauchs 13 erfolgt eine Vermessung des Fördervolumens dieses Schlauchs, beispielsweise indem die Flüssigkeitsmenge gemessen wird, die der Schlauch aufnehmen kann. Weicht das tatsächliche Fördervolumen des Schlauchs von dem Soll-Fördervolumen ab, wird eine entsprechende Kodierung an der Kodierplatte 22 angebracht, die das Maß der Abweichung angibt. Die Kodierung, die angebracht wird, wenn das Fördervolumen des Schlauchs gleich dem Soll-Fördervolumen ist, hat den Wert "0". Abweichungen des Fördervolumens von dem Soll-Fördervolumen werden entsprechend ihrer Größe kodiert, wobei aufgrund der zahlreichen Kodierschritte sehr feine Abstufungen möglich sind. Der Schlauch 13 wird, versehen mit seiner Kodierung, keimfrei verpackt und geliefert. Dieser Schlauch wird zur Benutzung in der dargestellten Weise an dem Infusionsgerät befestigt und sein oberes Ende wird mit einem Infusionslösungsbehälter 28 verbunden, aus dem die Flüssigkeit zum Patienten 29 gefördert werden soll.

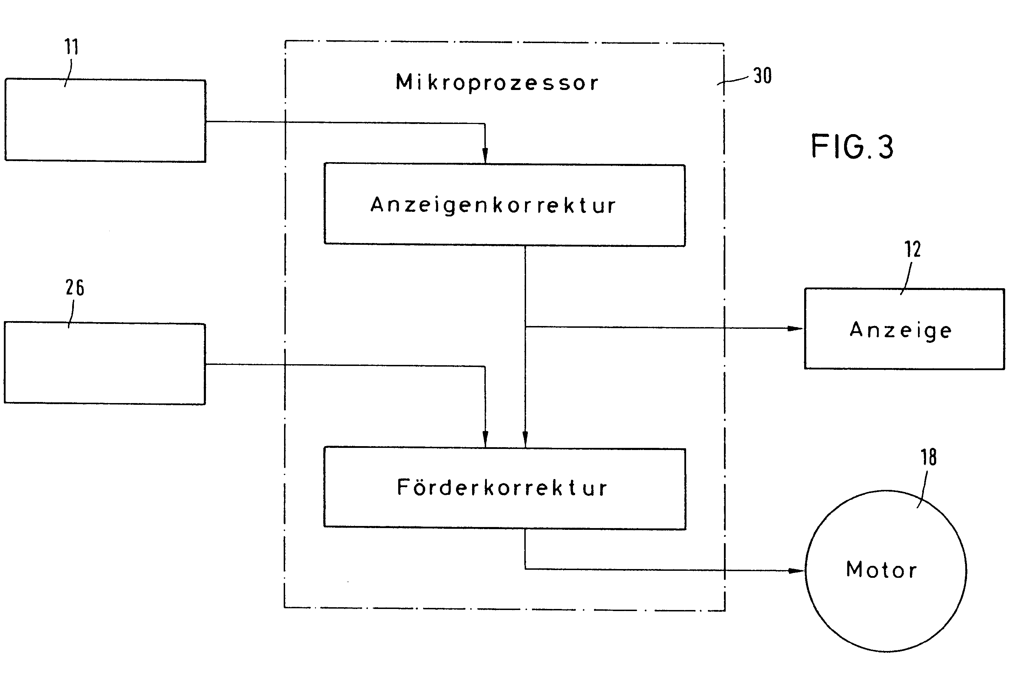

Der an der Tastatur 11 manuell eingestellte Wert für die Förderrate wird von dem Mikroprozessor 30 gemäß Fig. 3 verarbeitet und in Steuersignale für den Antrieb 18 umgesetzt. Im Mikroprozessor erfolgt zunächst eine Anzeigenkorrektur des eingegebenen Wertes. Der so gewonnene Wert wird an der Anzeigevorrichtung 12 als Soll-Förderrate angezeigt. Ferner wird der Korrekturwert, den die Kodierung repräsentiert, dem Mikroprozessor zugeführt und dieser korrigiert die dem Antrieb 18 zuzuführenden Steuersignale in der Weise, daß die individuellen Schlauchtoleranzen des betreffenden Schlauchs berücksichtigt werden und die Ist-Förderrate der angezeigten Soll-Förderrate entspricht.

## Patentansprüche

1. Medizinisches Pumpgerät mit einer in einem Gehäuse (10) untergebrachten Schlauchpumpe (14), deren Pumpenschlauch (13) auswechselbar eingesetzt werden kann und deren Antrieb (18) durch Steuersignale in Abhängigkeit von der eingestellten Förderrate gesteuert ist,
**dadurch gekennzeichnet**,
daß der Pumpenschlauch (13) mit einer Kodierung (22a) versehen ist, deren Kodiersignale das individuelle Fördervolumen dieses Schlauchs kennzeichnen, daß das Pumpgerät eine die Kodiersignale lesende Lesevorrichtung (26) aufweist und daß die den Antrieb (18) steuernden Steuersignale durch die gelesenen Kodiersignale entsprechend dem individuellen Fördervolumen des Schlauchs korrigiert werden.

2. Medizinisches Pumpgerät nach Anspruch 1, dadurch gekennzeichnet, daß das Gehäuse (10) eine Fixiervorrichtung (23) aufweist, die die Anbringung des Schlauchs (13) in nur einer definierten Position am Gehäuse zuläßt, in der die Kodierung (22a) von der Lesevorrichtung (26) erfaßt wird.

3. Medizinisches Pumpgerät nach Anspruch 1 oder 2, dadurch gekennzeichnet, daß die Schlauchpumpe (14) eine Fingerpumpe ist, die auf einer Seite des Schlauchs (13) mehrere den Schlauch an einem Gegenlager (17) abquetschende Pumpenfinger (15) aufweist, und daß die Lesevorrichtung (26) an einer Stelle des Gehäuses (10) angeordnet ist, an der das Gegenlager (17) den nicht abgequetschten Schlauch (13) überdeckt.

4. Medizinisches Pumpgerät nach einem der Ansprüche 1-3, dadurch gekennzeichnet, daß die Kodierung (22a) an einer am Schlauch (13) befestigten Kodierplatte (22) angeordnet ist, welche sich bei am Gehäuse (10) montiertem Schlauch gegen eine Gehäusewand (25) legt.

5. Medizinisches Pumpgerät nach einem der Ansprüche 1-4, dadurch gekennzeichnet, daß die Kodierung (22a) eine Binärkodierung ist.

## Claims

1. A medical pump device comprising a hose pump (14) arranged in a housing (10), the pump hose (13) of said hose pump (14) being adapted to be inserted in a manner allowing replacement thereof and the drive means (18) of said hose pump (14) being controlled by control signals in accordance with the set delivery rate,
**characterized in**
that the pump hose (13) has a coding (22a) provided thereon with its coding signals representing the individual delivery volume of the hose, that the pump device is provided with a reading means (26) for reading said coding signals and that the control signals for controlling the drive means (18) are corrected on the basis of the read coding signals in accordance with the individual delivery volume of the hose.

2. The medical pump device according to claim 1, characterized in that the housing (10) is provided with a fixing device (23) allowing attachment of the hose (13) on the housing in only one defined position in which the coding (22a) is detected by the reading means (26).

3. The medical pump device according to claim 1 or 2, characterized in that the hose pump (14) is a finger-type pump having a plurality of pump fingers (15) provided on one side of the hose (13) for squeezing the hose against an abutment (17), and that the reading means (26) is arranged at a position on the housing (10) where the abutment (17) covers the unsqueezed hose (13).

4. The medical pump device according to any one of claims 1 to 3, characterized in that the coding (22a) is provided on a coding plate (22) fastened to the hose (13), said coding plate (22) being disposed to lie against a housing wall (25) when the hose is mounted on the housing (10).

5. The medical pump device according to any one of claims 1 to 4, characterized in that the coding (22a) comprises a binary code.

## Revendications

1. Appareil médical à pompe comportant une pompe tubulaire (14), qui est logée dans un boîtier (10) et dont le tuyau souple (13) peut être inséré de façon interchangeable et dont le système d'entraînement (18) est commandé par des signaux de commande en fonction du débit réglé,
caractérisé en ce
que le tuyau souple (13) de la pompe est pourvu d'un codage (22a), dont les signaux de codage caractérisent le volume individuel de refoulement de ce tuyau souple, que l'appareil à pompe possède un dispositif de lecture (26) qui lit les signaux de codage et que les signaux de commande, qui commandent le dispositif d'entraînement (18), sont corrigés par les signaux de codage lus en fonction du volume individuel de refoulement du tuyau souple.

2. Appareil médical à pompe selon la revendication 1, caractérisé en ce que le boîtier (10) possède un dispositif de fixation (23), qui permet le montage du tuyau souple (13) sur le boîtier uniquement dans une position définie, dans laquelle le codage (22a) est détecté par le dispositif de lecture (26).

3. Appareil médical à pompe selon la revendication 1 ou 2, caractérisé en ce que la pompe tubulaire (14) est une pompe à doigts, qui comporte, sur un côté du tuyau souple (13), plusieurs doigts (15) qui comprime le tuyau souple contre une butée (17), et que le dispositif de lecture (26) est disposé en un emplacement du boîtier (10), auquel la butée (17) recouvre le tuyau souple (13) non comprimé.

4. Appareil médical à pompe selon l'une des revendications 1 à 3, caractérisé en ce que le codage (22a) est disposé sur une plaque de codage (22) qui est fixée sur le tuyau souple (13) et s'applique contre une paroi (25) du boîtier (10), lorsque le tuyau souple est monté sur ce dernier.

5. Appareil médical à pompe selon l'une des revendications 1 à 4, caractérisé en ce que le codage (22a) est un codage binaire.
